## Europäisches Patentamt
## European Patent Office
## Office européen des brevets

(11) Veröffentlichungsnummer: **0 366 908**
**A1**

## EUROPÄISCHE PATENTANMELDUNG

(21) Anmeldenummer: 89116740.5

(22) Anmeldetag: 09.09.89

(51) Int. Cl.5: **C07C 49/395**

(30) Priorität: 04.11.88 DE 3837452

(43) Veröffentlichungstag der Anmeldung:
09.05.90 Patentblatt 90/19

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Keil, Thomas, Dr.**
**Dormager Strasse 36**
**D-4370 Marl(DE)**
Erfinder: **Gull, Reinhold, Dr.**
**Leverkusener Strasse 29**
**D-4370 Marl(DE)**

(54) **Verfahren zur Herstellung von 2-Methyl-5-n-alkylcyclopentanonen.**

(57) Die bekannten Verfahren zur Herstellung von 2-Methyl-5-n-alkylcyclopentanonen gehen von konjugierten Dienen, Kohlenmonoxid und Wasser oder Alkoholen aus. Nachteilig ist das Arbeiten mit Fremdlösemitteln in hoher Verdünnung sowie die geringen Ausbeuten.

Es wurde nun gefunden, daß durch Abänderung der Hydroformylierungsbedingungen aus Olefinen, Kohlenmonoxid und Wasserstoff nicht Aldehyde, sondern cyclische Ketone entstehen, wenn man die Reaktion in Gegenwart einer Kobaltverbindung und ggf. tertiären Amins bei 100 bis 200 °C und 100 bis 350 bar durchführt.

Das Produkt findet insbesondere in der Riechstoffindustrie Anwendung.

EP 0 366 908 A1

## Verfahren zur Herstellung von 2-Methyl-5-n-alkylcyclopentanonen

Die Erfindung betrifft ein Verfahren zur Herstellung von 2-Methyl-5-n-alkylcyclopentanonen durch Reaktion von nicht konjugierten Polyenen mit Kohlenmonoxid und Wasserstoff bzw. Synthesegas an Kobalt-Katalysatoren.

Alkylcyclopentanone werden als Riechstoffe, als Zwischenprodukte für Riechstoffe, wie z. B. für die Herstellung von Lactonen, als Ausgangsmaterialien für pharmazeutische Produkte sowie als Vorprodukte für Lactame verwendet.

Nach US-PS 2 995 607 sind Fünfringketone durch Umsetzung von nicht konjugierten Dienen mit Kohlenmonoxid in Wasser als Lösungsmittel in Gegenwart von Kobaltkatalysatoren herstellbar. Die Reaktion soll in vollständiger Abwesenheit von Wasserstoff geführt werden, kleinste Mengen stören allerdings nicht.

Gemäß JA 61277-644 wird hierfür anstelle von Wasser ein Alkohol, z. B. Isopropanol, eingesetzt, als Katalysator wirken neben Kobaltverbindungen noch tertiäre Amine.

Nachteilig für diese Verfahren sind das Arbeiten mit Fremdlösemitteln in hoher Verdünnung sowie die geringen Ausbeuten.

Nach DE-OS 35 08 420 können 2-Alkylcyclopentanone auch durch Umsetzung von Cyclopentanonen mit Aldehyden in Gegenwart von Wasserstoff und einem Katalysator dargestellt werden. Als Katalysator werden Edelmetalle, wie z. B. Palladium, zusammen mit Oxiden von Titan, Aluminium oder Magnesium, eingesetzt.

Nach B. Fell, W. Seidel und F. Asinger (Tetrahydron Letters 8, 1003 (1968) erhält man 2.5-Dimethylcyclopentanon durch stöchiometrische Reaktion von 1.5-Hexadien mit Nickeltetracarbonyl in wäßriger Aceton lösung. Des weiteren kann man gemäß US-PS 2 863 923 2.5-Dimethylcyclopentanon durch Erhitzen von 2.5-Dimethyladipinsäuredimethylester über $MnO_2/Al_2O_3$ auf ca. 450 °C erhalten.

Aufgabe der vorliegenden Erfindung ist es, 2-Methyl-5-n-alkylcyclopentanone in wirtschaftlicher Weise und mit guten Ausbeuten herzustellen.

Diese Aufgabe wird erfindungsgemäß dadurch gelöst, daß man nicht konjugierte Polyene mit 5 bis 20 C-Atomen mit Kohlenmonoxid und Wasserstoff bzw. Synthesegas in Gegenwart einer Kobaltverbindung und gegebenenfalls eines tertiären Amins umsetzt.

Gegenstand der Erfindung ist daher ein Verfahren zur Herstellung von 2-Methyl-5-n-alkylcyclopentanonen durch Umsetzung von nicht konjugierten Polyenen mit Kohlenmonoxid und Wasserstoffaciden Komponenten, welches dadurch gekennzeichnet ist, daß man die Reaktion mit Wasserstoff oder Synthesegas in Gegenwart einer Kobaltverbindung und ggf. eines tertiären Amins bei 100 bis 200 °C und 100 bis 350 bar durchführt.

Die erfindungsgemäße Lösung ist überraschend, da Olefine unter den genannten Bedingungen zu den Aldehyden reagieren. Zwar ist bekannt, daß bei Umsetzungen von Kohlenmonoxid mit nicht konjugierten Dienen in Gegenwart H-acider Komponenten oder Wasserstoff cyclische Ketone als Nebenprodukte entstehen, doch ist es neu, durch Abänderungen der Hydroformylierungsbedingungen die Cyclopentanone als Hauptprodukte herstellen zu können.

Die Reaktion wird im allgemeinen in einem Autoklaven durchgeführt. Dabei wird vorzugsweise ein Druck von 150 bis 300 bar eingestellt, die Temperatur beträgt 120 bis 180 °C. Der Gesamtdruck resultiert aus einem Gemisch von Kohlenmonoxid und Synthesegas, wobei zu Beginn der Reaktion ein Molverhältnis von Kohlenmonoxid zu Synthesegas von 1:1,5 bis 25:1 vorgelegt wird. Das Molverhältnis Wasserstoff zu Kohlenmono xid soll dabei 0,02:1 bis 1:1 betragen. Der im Verlauf der Reaktion anfallende Druckverlust wird durch Nachdrücken von Synthesegas ausgeglichen.

Als Katalysator können Kobaltsalze, wie Kobalt-(II)-acetat, -naphthenat, -stearat, -carbonat oder -chlorid, Kobaltoxide oder Kobaltcarbonyle, wie Dikobaltoctacarbonyl, eingesetzt werden. Unter den gegebenen Reaktionsbedingungen von 120 bis 160 °C und 150 bis 300 bar bildet sich durch den Wasserstoff des Synthesegases Kobalttetracarbonylwasserstoff, das vermutlich die eigentliche aktive Verbindung ist. Die Bildung des Kobalttetracarbonylwasserstoffs ist im allgemeinen nach einer halben Stunde beendet. Bezogen auf das eingesetzte Polyen verwendet man vorzugsweise 0,5 bis 5 Mol-% Kobaltverbindung.

Bei Einsatz eines tertiären Amins als Promotor kommen Pyridin und nicht-ortho-substituierte Pyridine, wie z. B. 3- und 4-Picolin, 3.4-und 3.5-Lutidin und 3- und 4-Ethylpyridin, infrage. Das Molverhältnis tertiäres Amin : Kobaltverbindung beträgt 0,5:1 bis 10:1, vorzugsweise 1:1 bis 3:1.

Durch das erfindungsgemäße Verfahren wird ein allgemeiner Weg dafür aufgezeigt, wie 2-Methyl-5-n-alkylcyclopentanone in guten Ausbeuten aus nicht konjugierten Polyenen durch Reaktion mit Kohlenmonoxid und Wasserstoff hergestellt werden können.

Das Verfahren wird in Abwesenheit von fremden Lösungsmitteln durchgeführt. Es hat sich aber auch als vorteilhaft erwiesen, das Produkt selbst als Lösemittel zur verwenden.

Besonders eignen sich Polyene, deren Doppelbindungen in 1.4- oder 1.5-Stellung zueinander stehen und eine Doppelbindung endständig ist. Doch können auch Polyene mit größeren Abständen zwischen den Doppelbindungen eingesetzt werden, da der Kobaltkatalysator doppelbindungsisomerisierend wirken kann.

Beispiele

In den Beispielen, die die Erfindung verdeutlichen sollen, wird jeweils ein 5- bzw. 3 l-VA-Stahlautoklav mit Magnetrührer, Temperatur-und Druckregelung verwendet.

Die Reaktionskomponenten nicht konjugiertes Polyen, Kobaltverbindung und gegebenenfalls Amin werden eingefüllt und auf Reaktionstemperatur gebracht. Dann wird Kohlenmonoxid aufgedrückt, worauf durch Aufdrücken von Synthesegas der Gesamtdruck aufgebaut wird. Der Druckabfall während der Reaktion wird durch ständige Synthesegas-Zufuhr ausgeglichen.

Nach beendeter Reaktion wird auf Raumtemperatur abgekühlt und entspannt. Die Zusammensetzung des Reaktionsproduktes wird durch quantitative gaschromatografische Analyse bestimmt. Die Ketone wurden durch fraktionierte Destillation isoliert. In den Beispielen sind jeweils Mol-% Keton, bezogen auf eingesetztes Polyen, angegeben.

Beispiel 1

Einwaage

673,6 g (8,2 Mol) 1.5-Hexadien
25,9 g (0,33 Mol) Pyridin
96,6 g (0,16 Mol) Co-(II)-naphthenat ·

Reaktionsbedingungen:

$T = 140 °C$; $P_{co} = 190$ bar (anfangs), $P_{ges} = 250$ bar; $t = 12$ h
Bei einem Umsatz von > 99 %, bezogen auf eingesetztes 1.5-Hexadien, werden 53,3 % 2.5-Dimethylcyclopentanon erhalten.

Beispiel 2

Einwaage

1 564,8 g (14,2 Mol) 1.7-Octadien
67,4 g (0,85 Mol) Pyridin
167,4 g (0,28 Mol) Co-(II)-naphthenat

Reaktionsbedingungen:

$T = 140 °C$; $P_{co} = 160$ bar (anfangs), $P_{ges} = 200$ bar; $t = 18$ h
Bei einem Umsatz von > 99 %, bezogen auf eingesetztes 1.7-Octadien werden 34,3 % 2-Methyl-5-n-propylcyclopentanon erhalten.

Beispiel 3

Einwaage

1 586,9 g (14,4 Mol) 1.7-Octadien
22,8 g (0,29 Mol) Pyridin
169,7 g (0,29 Mol) Co-(II)-naphthenat

Reaktionsbedingungen:

$T = 140 °C$; $P_{co} = 160$ bar (anfangs), $P_{ges} = 200$ bar; $t = 24$ h
Bei einem Umsatz von > 99 %, bezogen auf eingesetztes 1.7-Octadien, werden 30,9 % 2-Methyl-5-n-propylcyclopentanon erhalten.

Beispiel 4

Einwaage

1 617,5 g (11,7 Mol) 1.9-Decadien
37,0 g (0,47 Mol) Pyridin
137,9 g (0,23 Mol) Co-(II)-naphthenat

Reaktionsbedingungen:

$T = 150 °C$; $P_{co} = 160$ bar (anfangs), $P_{ges} = 200$ bar; $t = 12$ h
Bei einem Umsatz von > 99 %, bezogen auf 1.9-Decadien, werden 16,8 % 2-Methyl-5-n-pentylcyclopentanon erhalten.

Beispiel 5

In diesem Beispiel wurde das Reaktionsprodukt (2-Methyl-5-n-propylcyclopentanon) als Lösungsmittel verwendet.

Einwaage

782,4 g (7,1 Mol) 1.7-Octadien
995,6 g (7,1 Mol) 2-Methyl-5-n-propylcyclopentanon
33,7 g (0,43 Mol) Pyridin

83,7 g (0,14 Mol) Co-(II)-naphthenat

Reaktionsbedingungen:

T = 140 °C; $P_{co}$ = 160 bar (anfangs), $P_{ges}$ = 200 bar; t = 12 h

Bei einem Umsatz von > 99 %, bezogen auf 1.7-Octadien werden 43,8 % 2-Methyl-5-n-propylcyclopentanon (insgesamt 10,2 Mol) erhalten.

Beispiel 6

Einwaage

591,5 g (7,2 Mol) 1.5-Hexadien
22,8 g (0,14 Mol) Pyridin
35,9 g (0,14 Mol) Co-(II)-acetat $\cdot$ 4 $H_2O$

Reaktionsbedingungen:

T = 130 °C; $P_{co}$ = 190 bar (anfangs), $P_{ges}$ = 250 bar; t = 12 h

Bei einem Umsatz von > 98 %, bezogen auf 1.5-Hexadien werden 52,9 % 2.5-Dimethylcyclopentanon erhalten.

Beispiel 7 (ohne Pyridin)

Einwaage

1 577,3 g (19,2 Mol) 1.5-Hexadien
226,3 g ( 0,384 Mol) Co-(II)-naphthenat

Reaktionsbedingungen:

T = 140 °C; $P_{co}$ = 190 bar (anfangs), $P_{ges}$ = 250 bar; t = 12 h

Bei einem Umsatz von 90 %, bezogen auf 1.5-Hexadien werden 45,3 % 2.5-Dimethylcyclopentanon erhalten.

Ansprüche

1. Verfahren zur Herstellung von 2-Methyl-5-n-alkylcyclopentanonen durch Umsetzung von nicht konjugierten Polyenen mit Kohlenmonoxid und Wasserstoff-aciden Komponenten,
dadurch gekennzeichnet,
daß man die Reaktion mit Wasserstoff oder Synthesegas in Gegenwart einer Kobaltverbindung und ggf. eines tertiären Amins bei 100 bis 200 °C und 100 bis 350 bar durchführt.

2. Verfahren nach Anspruch 1,
dadurch gekennzeichnet,
daß das Molverhältnis Wasserstoff : Kohlenmonoxid 0,02:1 bis 1:1 beträgt.

3. Verfahren nach den Ansprüchen 1 und 2,
dadurch gekennzeichnet,
daß das Molverhältnis Kohlenmonoxid zu Synthesegas zu Beginn der Reaktion 1:1,5 bis 25:1 beträgt und Druckverluste durch Nachdrücken von Synthesegas ausgeglichen werden.

4. Verfahren nach Anspruch 1,
dadurch gekennzeichnet,
daß die Reaktion bei 120 bis 180 °C durchgeführt wird.

5. Verfahren nach Anspruch 1,
dadurch gekennzeichnet,
daß die Reaktion bei einem Druck von 100 bis 300 bar durchgeführt wird.

6. Verfahren nach Anspruch 1,
dadurch gekennzeichnet,
daß man als Kobaltverbindung Salze, Oxide und/oder Komplexe des Kobalts einsetzt.

7. Verfahren nach Anspruch 1,
dadurch gekennzeichnet,
daß man als tert. Amine bevorzugt Pyridine, nicht-ortho-substituierte Alkylpyridine oder 4-Picolin verwendet.

8. Verfahren nach Anspruch 1,
dadurch gekennzeichnet,
daß die Kobaltverbindung, bezogen auf nichtkonjugiertes Polyen, in Mengen von 0,5 bis 5 Mol-% eingesetzt wird.

9. Verfahren nach Anspruch 1,
dadurch gekennzeichnet,
daß bei Einsatz eines tertiären Amins das Molverhältnis tertiäres Amin : Kobaltverbindung 0,5:1 bis 10:1 beträgt.

10. Verfahren nach Anspruch 1,
dadurch gekennzeichnet,
daß man nicht konjugierte Polyene mit 5 bis 20 C-Atomen einsetzt.

11. Verfahren nach Anspruch 1,
dadurch gekennzeichnet,
daß man vorzugsweise alpha-omega-Diene mit 6 bis 10 C-Atomen einsetzt.

**Europäisches Patentamt**

# EUROPÄISCHER RECHERCHENBERICHT

## EINSCHLÄGIGE DOKUMENTE

EP 89116740.5

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 5) |
|---|---|---|---|
| D,X | US - A - 2 995 607 (PETER P. KLEMCHUK) * Ansprüche * | 1,4,5, 10,11 | C 07 C 49/395 |
| D,X | PATENT ABSTRACTS OF JAPAN, unexamined applications, C Field, Band 11, Nr. 139, 7. Mai 1987 THE PATENT OFFICE JAPANESE GOVERNMENT Seite 101 C 420 * Kokai-Nr. 61-277 644 (NIPPON OIL) * | 1 | |
| A | US - A - 4 288 613 (RICHARD C. LAROCK) * Zusammenfassung * | 1 | |
| D,A | DE - A1 - 3 508 420 (BASF) * Zusammenfassung * | 1 | |
| A | US - A - 2 875 249 (HARRY A. STANSBURY et al.) * Spalten 1,2 - Zeile 45 * | 1 | |
| D,A | US - A - 2 863 293 (NEWMAN M. BORTNICK) * Anspruch 1 * | 1 | |
| A | EP - A1 - 0 093 853 (CHEMISCHE WERKE HÜLS) * Ansprüche * | 1 | |
| D,A | TETRAHEDRON LETTERS, Nr. 8, 1968 Pergamon Press, Oxford, London, New York, Paris BERNHARD FELL et al. "Über eine neue Synthese cyclischer | 1 | |

RECHERCHIERTE SACHGEBIETE (Int. Cl. 5)

C 07 C 49/00
C 07 C 45/00

Der vorliegende Recherchenbericht wurde für alle Patentansprüche erstellt.

| Recherchenort | Abschlußdatum der Recherche | Prüfer |
|---|---|---|
| WIEN | 05-02-1990 | REIF |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | Ketone bei der stöchiometrischen Hydrocarboxylierung von 1,5-Dienen mit Nickeltetracarbonyl" Seiten 1003-1006 * Seiten 1003-1004 * ---- | | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 05-02-1990 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82